# EUROPEAN PATENT SPECIFICATION

(11) **EP 1 902 712 B1**
(45) Date of publication and mention of the grant of the patent: **29.12.2010**
(21) Application number: 06742090.1
(22) Date of filing: 05.06.2006
(51) Int. Cl.: A61K 31/4168, A61P 33/00, A61K 9/00

(54) **USE OF LEVO-ORNIDAZOLE FOR PREPARING ANTIPARASITIC INFECTION DRUG**
VERWENDUNG VON LEVO-ORNIDAZOL ZUR HERSTELLUNG EINES ANTIPARASITÄREN INFEKTIONSWIRKSTOFFS
UTILISATION DU LÉVO-ORNIDAZOLE POUR LA PRÉPARATION DE MÉDICAMENTS CONTRE LES INFECTIONS PARASITAIRES

(30) Priority: 08.07.2005 CN 200510083517
(43) Date of publication of application: 26.03.2008
(73) Proprietor: Nanjing Sanhome Pharmaceutical Co., Ltd., Nanjing Jiangsu 210038 (CN)
(72) Inventor: ZHANG, Cang, Jiangsu 210038 (CN); TAO, Xiaoxin, Jiangsu 210038 (CN)
(74) Representative: Smaggasgale, Gillian Helen
(86) International application number: PCT/CN2006/001204
(87) International publication number: WO 2007/006197

(56) References cited:
- CN-A- 1 390 836
- CN-A- 1 400 312
- ANONYMUS: "Tiberal - Alkophar" FACHINFORMATION DES ARZNEIMITTEL-KOMPENDIUM IN DER SCHWEIZ, [Online] November 2004 (2004-11), pages 1-5, XP007905192 Retrieved from the Internet: URL:http://www.kompendium.ch/MonographieTx t.aspx?lang=de&MonType=fi> [retrieved on 2008-07-15]
- DATABASE WPI Week 200618 Thomson Scientific, London, GB; AN 2006-165583 XP002489635 -& CN 1 686 116 A (NANJING SHENGHE PHARM CO LTD) 26 October 2005 (2005-10-26)
- DATABASE CA [Online] BONE W. ET AL.: 'Toxicity of ornidazole and its analogs to rat spermatozoa as reflected in motility parameters', XP003006285 Database accession no. (129:49760) & INT. J. ANDROL. vol. 20, no. 6, 1997, pages 347 - 355
- DATABASE CA [Online] SKUPIN R. ET AL.: 'Lipase-catalyzed resolution of both enantiomers of ornidazole and some analogs', XP003006286 Database accession no. (127:220606) & TETRAHEDRON: ASYMMETRY vol. 8, no. 14, 1997, pages 2453 - 2464

## Description

### FIELD OF THE INVENTION

This invention relates to the use of Levo-ornidazole in the preparation of anti-parasitic infection drug, and particularly to the drug preparations prepared by formulating Levo-ornidazole into anti-parasitic infection drugs suitable for clinical use, especially for trichomonas vaginalis infection and cecal amoeba infection. Preferred preparations include oral preparation, intravenous preparation and vaginal preparation.

### BACKGROUND OF THE INVENTION

Levo-ornidazole (1-(3-chloro-2-S-(-)-hydroxypropyl)-2-methyl-5-nitroimidazole) is the levo-isomer of ornidazole (CAS 16773-42-5). As a nitroimidazole derivative, ornidazole is a powerful anti-anaerobic bacteria and anti-parasite infection agent, and also as the newly developed third-generation of nitroimidazole derivative next to the 2-methyl-5-nitro-1H-Imidazole-1-ethanol, ornidazole exhibits higher therapeutical efficacy, shorter clinical course, better tolerance, and wider in-vivo distribution. The anti-microorganisms effect of ornidazole is promoted by the reduction of nitro group of its molecule to amino group under anaerobic environment, or by the formation of free radical followed by interaction with the cellular components, and caused to the death of microorganisms. The usefulness of ornidazol in the treatment of parasitic infections is disclosed in "Tiberal - Alkopliar "FACHINFORMATION DES ARZNEIMTTEL-KOMPENDIUM IN DER SCHWEIZ" Ornidazole racemate is the principal component in commercial ornidazole preparations. In China, there is a patent application (CN 1400312A) regarding the separation of racemic ornidazole into L- and D-ornidazole by means of enzymatic resolution, however, comparative studies on the pharmacology and pharmacodynamics among L- and D-ornidazole and racemic ornidazole have not been published yet.

### DISCLOSURE OF THE INVENTION

Clinical use of ornidazole shows that ornidazole is effective in treating anaerobic bacteria infections, but there also are some adverse reactions. Research studies on L-ornidazole have been conducted by the inventor of the present invention with respect to its pharmacokinetics, pharmacodynamics, toxicology and general pharmacology, in which 1-ornidazole is found having pharmacokinetics characteristics superior to D-ornidazole and racemic ornidazole, and also having lower central nervous system toxicity than D-ornidazole and racemic ornidazole.

From the studies on acute toxicology, it was found that in the case of administration of L-ornidazole in mice. LD₅₀ was 332mg/kg (95% Cl: 312~362mg/kg) for intravenous injection, 1378mg/kg (95% Cl: 1244~1526mg/kg) for intraperitoneal injection and 1069mg/kg (95%) Cl: 935.3~1222mg/kg) for oral gavage. In the case of racemic ornidazole, LD₅₀ was 306mg/kg (95% Cl: 272~346mg/kg) for intravenous injection, 1115mg/kg (95% Cl: 1026~1212mg/kg) for intraperitoneal injection and 769.4mg/kg (95% Cl: 674.2~878.0mg/kg) for oral gavage. In accordance with the above results, it was demonstrated that L-ornidazole exhibited lower toxicity and relatively higher safety as compared with the racemic ornidazole.

For the toxicity test, beagle dogs (non-rodent) were administered intravenously the L-, D- and racemic ornidazole for two weeks and the results showed that L-ornidazole exhibited lower central toxicity and relatively higher safety as compared with D-ornidazole and racemic ornidazole.

General pharmacology of L-, D- and racemic ornidazole on central nervous system in mice was studied. The results suggested that L-ornidazole exhibited less inhibitory effect on the central nervous system as compared with D- or racemic ornidazole.

Based on the above experiments, pharmacodynamic studies were conducted on the therapeutical efficacy of L-ornidazole in treating parasitic infections (including trichomonas vaginalis infection and cecal amoeba infection in mice). The results showed that L-ornidazole was superior to D-ornidazole and racemic ornidazole in the therapeutical efficacy for treating parasitic infections (including trichomonas vaginalis infection and cecal amoeba infection in mice). The detailed experimental procedures were as follows:
(I) Pharmacodynamic studies on trichomonas vaginalis infection
0.4ml solution containing 3 × 10⁶ trichomonas vaginalis (trichomonas vaginalis taken from clinical patients) was administered to each male ICR mouse by intraperitoneal injection. The mice were randomly divided into 19 groups, 10 mice in each group. Blank solutions were administered to the mice in vehicle control group by tail intravenous injection) while active drugs were administered to the treatment groups by intravenous injection 2. 24, 48, 72 hours after infection. The animals were killed five days after infection, and their offal was washed, and washing water thereof was centrifuged for the examination of any presence of live larvae. Autopsy was performed in examining the condition of visceral and abdominal abscess, and the number of live trichomoniasis in the abscess was determined under microscope. The results showed that multiple small abscess were formed in the abdomen and offal of mice in the vehicle control group while abscess formation and the number of live trichomoniasis was reduced in the treatment groups was inhibited. The dosages required to achieve the 50% (ED₅₀) and 90% (ED₉₀) inhibition rate were calculated and the results were shown in Table 1.

**Table 1. Pharmacodynamic studies on trichomonas vaginalis infection**

| Drug | ED₅₀ | 95% Cl | ED₉₀ | 9.5% Cl |
|---|---|---|---|---|
| L-ornidazole | 9.1 | 4.8~17.4 | 32.6 | 17.0~61.7 |
| D-ormidazole | 14.1 | 8.3∼24.0 | 54.5 | 32.4~93.3 |
| Racemic ornidazole | 11.3 | 5.5 ~22.9 | 43.8 | 21.4~89.1 |

The above experimental results showed that L-ornidazole was superior to D-ornidazole and racemic ornidazole in the therapeutical efficacy for treating trichomonas vaginalis infections in mice.
(II) Pharmacodynamic studies on the cecal amoeba infection
0.2 ml solution containing 200,000 units of amebic dysentery was administered to each male ICR mouse at the cecum by injection. The mice were randomly divided into 19 groups, 10 mice in each group. Blank solutions were administered to the mice in vehicle control group by tail intravenous injection while active drugs were administered to the treatment groups by intravenous injection 2, 24, 48, 72 hours after infection. The animals were killed six days after infection. Intestinal mucosal sections were made, and comparative studies on amoeba of different stages were performed under microscope. The results showed the growth of amebic dysentery in the cecum in the vehicle control group, and the inhibition and killing of the amebic dysentery in the treatment groups. The dosages required to achieve the 50% (ED₅₀) and 90% (ED₉₀) inhibition rate were calculated and the results were shown in Table 2.

**Table 2. Pharmacodynamic studies on cecal amoeba infection**

| Drug | ED₅₀ | 95% Cl | ED₅₀ | 95% Cl |
|---|---|---|---|---|
| L-ornidazole | | 5.4~20.4 | 41.2 | 21.4~81.3 |
| D-ornidazole | 13.3 | 8.3~20.8 | 64.4 | 40.7~102.3 |
| Racemic ornidazole | 11.4 | 5.6~22.9 | 55.2 | 27.5~109.6 |

The above experimental results showed that L-ornidazole was superior to D-ornidazole and racemic ornidazole in the therapeutical efficacy for treating cecal amoeba infection in mice.

These experiments show that L-ornidazole was superior to D-ornidazole and racemic ornidazole in the therapeutical efficacy for treating parasitic infections (including trichomonas vaginalis infection and cecal amoeba infection in mice). In addition, pharmacokinetics characteristics of L-ornidazole were superior to that of D- and racemic ornidazole, and L-ornidazole exhibited lower toxicity and less central nervous inhibitory effects than D- or racemic ornidazole. For these reasons, it would be more practicable to formulate L-ornidazole as anti-parasitic infection drugs.

The present invention also provides drug preparations that contain L-ornidazole as the principal component. Preferably, said preparations include oral preparations, intravenous preparations or vaginal preparations. The dosage of the oral preparations according to the present invention is preferably 10~40mg/kg/day, and more preferably 20~30mg/kg/day. The dosage of the intravenous preparations according to the present invention is preferably 5~40mg/kg/day, and more preferably 10~20mg/kg/day. The dosage of the vaginal preparations according to the present invention is preferably 10~40mg/kg/day, and more preferably 20~30mg/kg/day.

The following examples are given for the purpose of illustrating the present invention.

### EXAMPLE 1

### Formulation:

| Ingredients | Quantity(mg/tablet) |
|---|---|
| L-ornidazole | 250 |
| Pregelatinized Starch | 80 |
| Sodium Starch Glycolate | 4 |
| Magnesium stearate | 3 |

For exemplification, 1000 tablets were prepared. Specifically, the active ingredient and the adjuvants were sieved through a 100-mesh sieve. Prescribed amount of L-ornidazole and pregelatinized starch were weighed and mixed thoroughly, followed by addition of 8% starch slurry to prepare the damp mass, which was then subjected to granulating, drying and sizing. To the dry granules were added prescribed amount of sodium starch glycolate and magnesium stearate. Subsequently, the granules were compressed and coated by 8% opadry in 95% ethanol solution.

### EXAMPLE 2

### Formulation:

| Ingredients | Quantity(mg/capsule) |
|---|---|
| L-ornidazole | 250 |
| Starch | 45 |
| Magnesium stearate | 2 |

For exemplification, 1000 capsules were prepared. Specifically, the active ingredient and the adjuvants were sieved through a 100-mesh sieve. Prescribed amount of L-ornidazole and starch were weighed and mixed thoroughly, followed by addition of 6% starch slurry to prepare the damp mass, which was then subjected to granulating, drying and sizing. To the dry granules were added prescribed amount of magnesium stearate, mixed thoroughly and filled up the capsules.

### EXAMPLE 3

### Formulation:

| Ingredients | Quantity(mg/bag) |
|---|---|
| L-ornidazole | 250 |
| Mannitol | 250 |
| Sucrose | 200 |
| Sodium Starch Glycolate | 20 |

For exemplification, 1000 bags were prepared. Specifically, the active ingredient the adjuvants were sieved through a 100-mesh sieve. Prescribed amount of L-ornidazole, mannitol, sucrose and sodium starch glycolate were weighed and mixed thoroughly, followed by addition of 8% starch slurry to prepare the damp mass, which was then subjected to granulating, drying, sizing, and packing.

### EXAMPLE 4

### Formulation:

| Ingredients | Quantity |
|---|---|
| L-ornidazole | 5 mg/ml |
| Sodium Chloride | 8.30 mg/ml |
| Injection water (added up to) | 100ml |

For exemplification, 100 bottles of L-ornidazole sodium chloride injection were prepared. Specifically, prescribed amount of L-ornidazole and sodium chloride were weighed, followed by addition of 8L injection water of 40°C, stirred and dissolved. The pH of the solution was adjusted to 4.0 by 0.1mol/l hydrochloric acid, and the solution was added with injection water of 40°C to the required total volume. Subsequently, to the resultant solution, 0.1%, active carbon was added. The solution was stirred and left to stand for 15 minutes followed by decarburization with a titanium bar (5µm). For further filtration, the solution was passed through the microvoid filter films (0.45µm and a 0.22µm) of a filter cartridge. The resultant solution was filled and scaled in 100ml glass infusion bottles, which were then subjected to sterilization in a flowing stream of 100°C for 45 minutes.

### EXAMPLE 5

### Formulation:

| Ingredients | Quantity |
|---|---|
| L-ornidazole | 5mg/ml |
| Glucose | 50mg/ml |
| Injection water (added up to) | 100ml |

For exemplification, 100 bottles of L-ornidazole and glucose injection were prepared. Specifically, prescribed amount of L-ornidazole and glucose were weighed, and dissolved in 8L injection water of 45°C. The pH of the solution was adjusted to 3.5 by 0.1 mol/L hydrochloric acid, and the solution was added with injection water of 45°C to the required total volume. Subsequently, to the resultant solution, 0.15% active carbon was added. The solution was stirred and left to stand for 15 minutes, followed by decarburization with a titanium bar (5µm). For further filtration, the solution was passed through the microvoid filter films (0.45µm and a 0.22µm) of a filter cartridge. The resultant solution was filled and sealed in 100ml glass infusion bottles, which were then subjected to sterilization in a flowing stream of 100°C for 45 minutes to give the L-ornidazole and glucose injection.

### EXAMPLE 6

### Formulation:

| Ingredients | Quantity |
|---|---|
| L-ornidazole | 25mg/ml |
| Propylene glycol 10ml | 0.5ml/ml |
| Injection water (added up to) | 10ml |

For exemplification, 100 bottles of L-ornidazole injection were prepared. Specially, prescribed amount of L-ornidazole was weighed, and dissolved in propylene glycol of about 45°C. followed by addition of 100mL injection water of 45 °C and stirred. The pH of the solution was adjusted to 4.5 by 0.1mol/L. hydrochloric acid. After dissolution, and the solution was added with injection water of 45°C to the required total volume. Subsequently, to the resultant solution, 0.1% active carbon (for injection use) was added. The solution was stirred and left to stand for 15 minutes, followed by decarburization with a titanium bar (5µm). For further filtration, the solution was passed through the microvoid filter films (0.45µm and a 0.22µm) of a filter cartridge. The resultant solution was filled and sealed in ampoule bottles, which were then subjected to sterilization in a flowing stream of 100°C for 45 minutes to give the L-ornidazole injection.

### EXAMPLE 7

### Formulation:

| Ingredients | Quantity(mg/tablet) |
|---|---|
| L-ornidazole | 500 |
| Sodium bicarbonate | 300 |
| Low-substituted hydroxypropyl cellulose | 100 |
| Sodium lauryl sulfate | 3.2 |
| Microcrystalline cellulose | 440 |
| Tartaric acid | 280 |
| Polyethylene Glycol | 16 |

For exemplification, 100 vaginal effervescent tablets were prepared. Specifically, the active ingredient and the adjuvants were sieved through an 80-mesh sieve. Prescribed amount or L-ornidazole, sodium lauryl sulfateand, sodium bicarbonate. 75% prescribed amount of microcrystalline cellulose and 80% prescribed amount of low-substituted hydroxypropyl cellulose were weighed and mixed thoroughly, followed by addition of 60% ethanol to prepare the damp mass, which was then subjected to granulating using a 14-mesh nylon sieve, and wet granulate were dried in an oven at 60°C until the moisture content become <1.0%, followed by sizing using a 12-mesh nylon sieve to afford granule A: prescribed amount of organic acid and the rest of microcrystalline cellulose, low-substituted hydroxypropyl cellulose remained were weighed and mixed thoroughly, followed by addition of 60% ethanol to prepare the damp mass, which was then subjected to granulating using a 14-mesh nylon sieve, and left drying in an oven at 60°C until the moisture content become ≤1.5%, followed by sizing using a 12-mesh nylon sieve to afford granule B: granule A and granule B were mixed thoroughly with polyethylene glycol, and the resultant mixture was compressed using a heterogeneous punch and then packed to afford the vaginal effervescent tablets

## Claims

1. L-ornidazole for use in the treatment of parasitic infections.

2. L-ornidazole for use according to claim 1, **characterized in that** L-ornidazole is formulated into anti-parasitic infection pharmaceutical preparations for clinical use including oral preparation, intravenous preparation and vaginal preparation.

3. L-ornidazole for use according to claim 2, **characterized in that** the dosage of oral preparation is preferably 10-40mg/kg/day, and more preferably 20-30mg/kg/day.

4. L-ornidazole for use according to claim 2, **characterized in that** the dosage of intravenous preparation is preferably 5-40 mg/kg/day, and more preferably 10-20mg/kg/day.

5. L-ornidazole for use according to claims 1 to 2, **characterized in that** the dosage of vaginal preparation is preferably 10-40mg/kg/day, and more preferably 20-30mg/kg/day.

6. L-ornidazole for use according to any one of claims 1 to 4, **characterized in that** said parasitic infection is trichomonas vaginalis infection.

7. L-ornidazole for use according to any one of claims 1 to 4, **characterized in that** said parasitic infection is cecal amoeba infection.

8. Use of L-ornidazole in the preparation of an anti-parasitic infection medicament.

9. Use according to claim 8, **characterized in that** L-ornidazole is formulated into anti-parasitic infection pharmaceutical preparations for clinical use including oral preparation, intravenous preparation and vaginal preparation.

10. Use according to claim 9, **characterized in that** the dosage of oral preparation is preferably 10-40mg/kg/day, and more preferably 20-30mg/kg/day.

11. Use according to claim 9, **characterized in that** the dosage of intravenous preparation is preferably 5-40 mg/kg/day, and more preferably 10-20mg/kg/day.

12. Use according to claim 9, **characterized in that** the dosage of vaginal preparation is preferably 10-40mg/kg/day, and more preferably 20-30mg/kg/day.

13. Use according to any one of claims 8 to 11, **characterized in that** said parasitic infection is trichomonas vaginalis infection.

14. Use according to any one of claims 8 to 11, **characterized in that** said parasitic infection is cecal amoeba infection.

## Patentansprüche

1. L-Ornidazol zur Verwendung bei der Behandlung parasitischer Infektionen.

2. L-Ornidazol zur Verwendung nach Anspruch 1, **dadurch gekennzeichnet, daß** L-Ornidazol zu pharmazeutischen Präparaten einschließlich oralen, intravenösen und vaginalen Präparaten gegen parasitische Infektion zur klinischen Anwendung formuliert ist.

3. L-Ornidazol zur Verwendung nach Anspruch 2, **dadurch gekennzeichnet, daß** die Dosierung des oralen Präparats vorzugsweise 10 bis 40 mg/kg/Tag und insbesondere 20 bis 30 mg/kg/Tag ist.

4. L-Ornidazol zur Verwendung nach Anspruch 2, **dadurch gekennzeichnet, daß** die Dosierung des intravenösen Präparats vorzugsweise 5 bis 40 mg/kg/Tag und insbesondere 10 bis 20 mg/kg/Tag ist.

5. L-Ornidazol zur Verwendung nach Anspruch 1 bis 2, **dadurch gekennzeichnet, daß** die Dosierung des vaginalen Präparats vorzugsweise 10 bis 40 mg/kg/Tag und insbesondere 20 bis 30 mg/kg/Tag ist.

6. L-Ornidazol zur Verwendung nach einem der Ansprüche 1 bis 4, **dadurch gekennzeichnet, daß** die parasitische Infektion die Infektion Trichomonas vaginalis ist.

7. L-Ornidazol zur Verwendung nach einem der Ansprüche 1 bis 4, **dadurch gekennzeichnet, daß** die parasitische Infektion zökale Amöbeninfektion ist.

8. Verwendung von L-Ornidazol in der Zubereitung eines Medikaments gegen parasitische Infektion.

9. Verwendung nach Anspruch 8, **dadurch gekennzeichnet, daß** L-Ornidazol zu pharmazeutischen Präparaten einschließlich oralen, intravenösen und vaginalen Präparaten gegen parasitische Infektion zur klinischen Anwendung formuliert ist.

10. Verwendung nach Anspruch 9, **dadurch gekennzeichnet, daß** die Dosierung des oralen Präparats vorzugsweise 10 bis 40 mg/kg/Tag und insbesondere 20 bis 30 mg/kg/Tag ist.

11. Verwendung nach Anspruch 9, **dadurch gekennzeichnet, daß** die Dosierung des intravenösen Präparats vorzugsweise 5 bis 40 mg/kg/Tag und insbesondere 10 bis 20 mg/kg/Tag ist.

12. Verwendung nach Anspruch 9, **dadurch gekennzeichnet, daß** die Dosierung des vaginalen Präparats vorzugsweise 10 bis 40 mg/kg/Tag und insbesondere 20 bis 30 mg/kg/Tag ist.

13. Verwendung nach einem der Ansprüche 8 bis 11, **dadurch gekennzeichnet, daß** die parasitische Infektion die Infektion Trichomonas vaginalis ist.

14. Verwendung nach einem der Ansprüche 8 bis 11, **dadurch gekennzeichnet, daß** die parasitische Infektion zökale Amöbeninfektion ist.

## Revendications

1. L-ornidazole pour son utilisation dans le traitement d'infections parasitaires.

2. L-ornidazole pour son utilisation selon la revendication 1, **caractérisé en ce que** le L-ornidazole est formulé dans des préparations pharmaceutiques antiparasitaires pour une utilisation clinique incluant une préparation orale, une préparation intraveineuse et une préparation vaginale.

3. L-ornidazole pour son utilisation selon la revendication 2, **caractérisé en ce que** la posologie de la préparation orale est de préférence de 10 à 40 mg/kg/jour, et de manière davantage préférée de 20 à 30 mg/kg/jour.

4. L-ornidazole pour son utilisation selon la revendication 2, **caractérisé en ce que** la posologie de la préparation intraveineuse est de préférence de 5 à 40 mg/kg/jour, et de manière davantage préférée de 10 à 20 mg/kg/jour.

5. L-ornidazole pour son utilisation selon la revendication 2, **caractérisé en ce que** la posologie de la préparation vaginale est de préférence de 10 à 40 mg/kg/jour, et de manière davantage préférée de 20 à 30 mg/kg/jour.

6. L-ornidazole pour son utilisation selon l'une quelconque des revendications 1 à 4, **caractérisé en ce que** ladite infection parasitaire est une infection par trichomonas vaginalis.

7. L-ornidazole pour son utilisation selon l'une quelconque des revendications 1 à 4, **caractérisé en ce que** ladite infection parasitaire est l'amibiase caecale.

8. Utilisation de L-ornidazole dans la préparation d'un médicament antiparasitaire.

9. Utilisation selon la revendication 8, **caractérisée en ce que** le L-ornidazole est formulé dans des préparations pharmaceutiques antiparasitaires pour une utilisation clinique incluant une préparation orale, une préparation intraveineuse et une préparation vaginale.

10. Utilisation selon la revendication 9, **caractérisée en ce que** la posologie de la préparation orale est de préférence de 10 à 40 mg/kg/jour, et de manière davantage préférée de 20 à 30 mg/kg/jour.

11. Utilisation selon la revendication 9, **caractérisée en ce que** la posologie de la préparation intraveineuse est de préférence de 5 à 40 mg/kg/jour, et de manière davantage préférée de 10 à 20 mg/kg/jour.

12. Utilisation selon la revendication 9, **caractérisée en ce que** la posologie de la préparation vaginale est de préférence de 10 à 40 mg/kg/jour, et de manière davantage préférée de 20 à 30 mg/kg/jour.

13. Utilisation selon l'une quelconque des revendications 8 à 11, **caractérisée en ce que** ladite infection parasitaire est une infection par trichomonas vaginalis.

14. Utilisation selon l'une quelconque des revendications 8 à 11, **caractérisée en ce que** ladite infection parasitaire est l'amibiase caecale.
